# FASCICULE DE BREVET EUROPEEN

(11) **EP 2 202 225 B1**
(45) Date de publication et mention de la délivrance du brevet: **24.10.2012**
(21) Numéro de dépôt: 09290990.2
(22) Date de dépôt: 23.12.2009
(51) Int. Cl.: C07D 223/16, A61K 31/55, A61P 9/00

(54) **Nouveau procédé de synthèse de l'ivabradine et de ses sels d'addition à un acide pharmaceutiquemenet acceptable**
Neues Syntheseverfahren von Ivabradin und seinen Salzen als Zusatz zu einer pharmazeutisch akzeptierbaren Säure
New method for synthesising ivabradine and its added salts with a pharmaceutically acceptable acid.

(30) Priorité: 24.12.2008 FR 0807444
(43) Date de publication de la demande: 30.06.2010
(73) Titulaire: Les Laboratoires Servier, 92284 Suresnes Cedex (FR)
(72) Inventeur: Peglion, Jean-Louis, 78110 Le Vesinet (FR); Dessinges, Aimée, 92500 Rueil Malmaison (FR); Serkiz, Bernard, 77170 Servon Brie Comte Robert (FR); Lerestif, Jean-Michel, 76190 Yvetot (FR); Lecouve, Jean-Pierre, 76600 Le Havre (FR)

(56) Documents cités:
- WO-A-2008/065681
- WO-A-2008/146308

## Description

La présente invention concerne un procédé de synthèse de l'ivabradine de formule (I) : 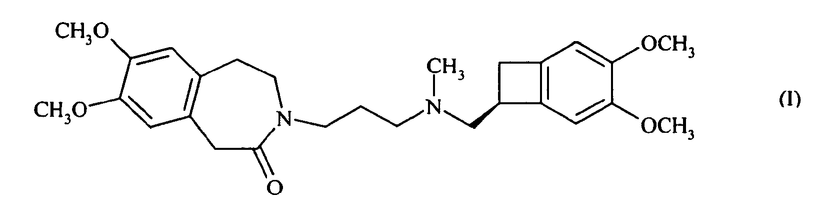 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de leurs hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires, et dans l'insuffisance cardiaque.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0 534 859.

Ce brevet décrit la synthèse du chlorhydrate de l'ivabradine à partir du composé de formule (II) : 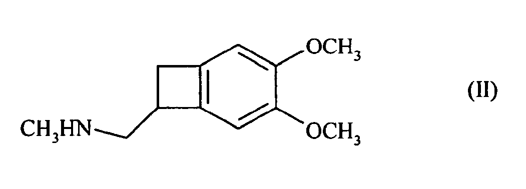 qui est dédoublé pour conduire au composé de formule (III) : 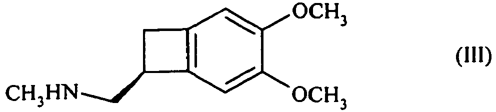 qui est mis en réaction avec le composé de formule (IV) : 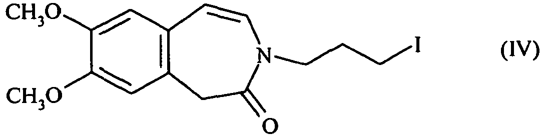 pour conduire au composé de formule (V) : 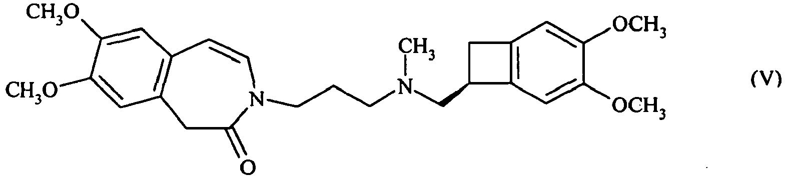 dont l'hydrogénation catalytique conduit à l'ivabradine, qui est alors transformée en son chlorhydrate.

L'inconvénient de cette voie de synthèse est de ne conduire à l'ivabradine qu'avec un rendement de 1 %.

Des procédés de synthèse alternatifs de l'ivabradine, basés également sur des réactions d'alkylation, ont été décrits dans les demandes internationales WO 2008/146 308 et WO 2008/065 681.

Compte-tenu de l'intérêt pharmaceutique de ce composé, il était important de pouvoir y accéder avec un procédé de synthèse performant, conduisant à l'ivabradine avec un bon rendement.

La présente invention concerne un procédé de synthèse du composé de formule (VI), sous forme racémique ou optiquement active : 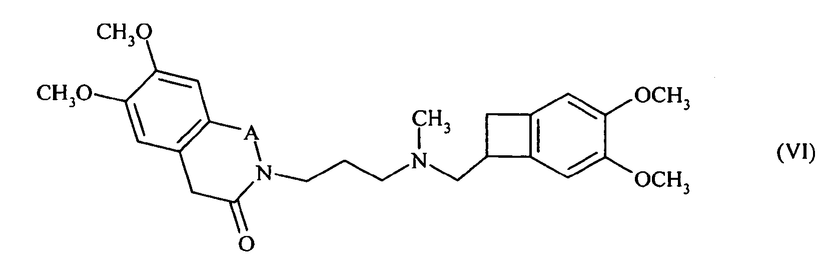 dans laquelle A représente H₂C-CH₂ ou HC=CH,
**caractérisé en ce que** l'on soumet le composé de formule (VII), sous forme racémique ou optiquement active : 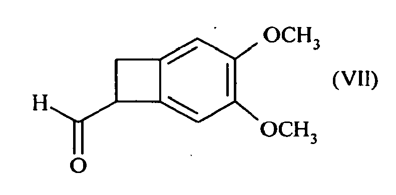 à une réaction d'amination réductrice par le composé de formule (VIII) : 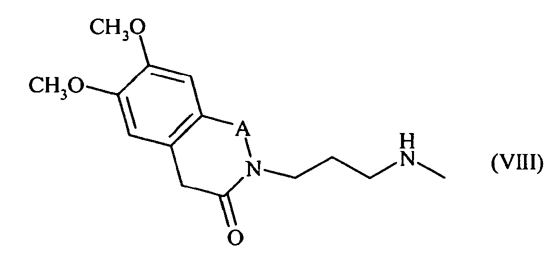 dans laquelle A est tel que défini précédemment,
en présence d'un agent de réduction,
dans un solvant organique ou un mélange de solvants organiques.

Dans un mode de réalisation préféré de l'invention, le composé de formule (VII) est sous forme optiquement active, et plus particulièrement de configuration (*S*).

Dans le cas où A représente H₂C-CH₂, le produit de l'amination réductrice du composé de formule (VII) de configuration (*S*) par le composé de formule (VIII) est l'ivabradine de formule (I) : 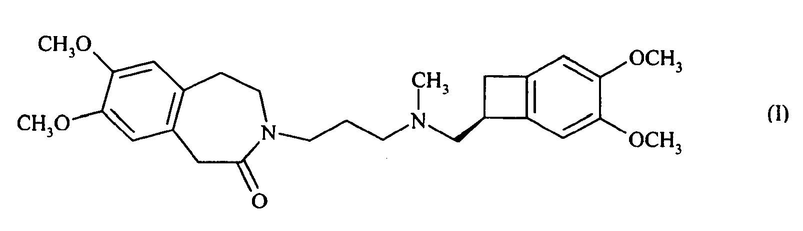 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Dans le cas où A représente HC=CH, le produit de l'amination réductrice du composé de formule (VII) de configuration (*S*) par le composé de formule (VIII) est le composé de formule (V) : 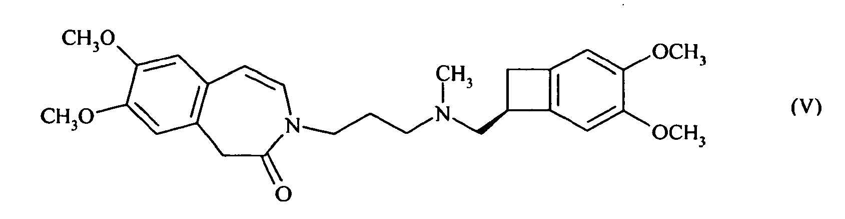 dont l'hydrogénation catalytique conduit à l'ivabradine de formule (I) : 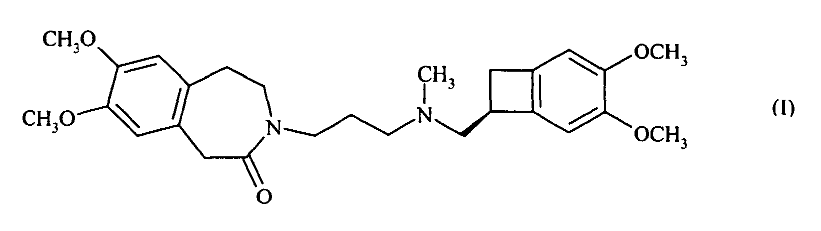 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Dans un autre mode de réalisation préféré de l'invention, le composé de formule (VII) est sous forme racémique. La réaction d'amination réductrice du composé de formule (VII) racémique par le composé de formule (VIII) est alors suivie d'une étape de résolution optique du composé de formule (VI) obtenu.

Dans le cas où A représente H₂C-CH₂, le produit obtenu après l'étape de résolution optique du composé de formule (VI) est l'ivabradine de formule (I) : 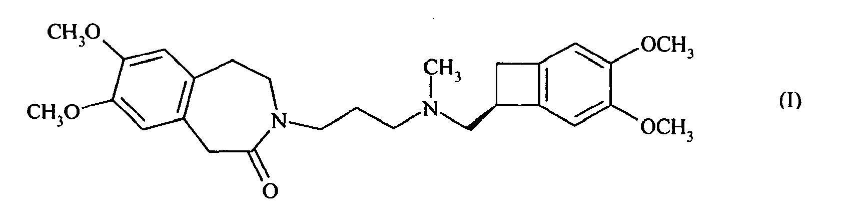 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Dans le cas où A représente HC=CH, le produit obtenu après l'étape de résolution optique du composé de formule (VI) est le composé de formule (V) : 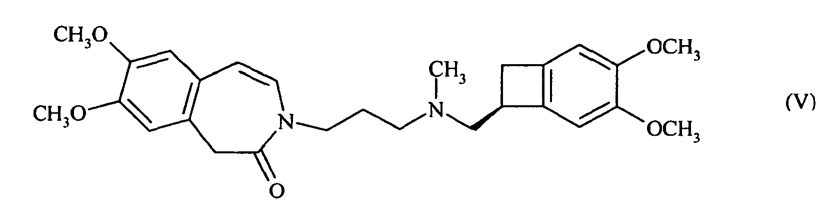 dont l'hydrogénation catalytique conduit à l'ivabradine de formule (I) : 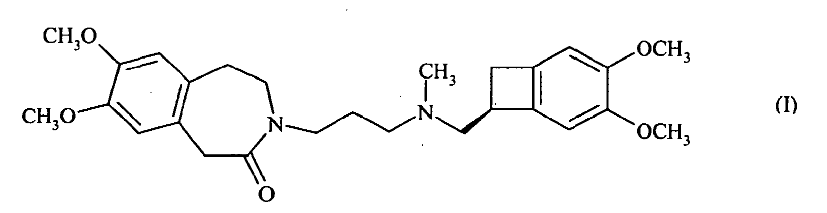 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

Parmi les agents de réduction pouvant être utilisés pour effectuer la réaction d'amination réductrice du composé de formule (VII) par le composé de formule (VIII), on peut citer à titre non limitatif les composés donneurs d'hydrures ou le dihydrogène en présence d'un catalyseur.

Parmi les agents de réduction pouvant être utilisés pour effectuer la réaction d'amination réductrice du composé de formule (VII) par le composé de formule (VIII), on peut citer à titre non limitatif le triacétoxyborohydrure de sodium, le cyanoborohydrure de sodium et le dihydrogène en présence d'un catalyseur tel que le palladium, le platine, le nickel, le ruthénium, le rhodium, ainsi que leurs dérivés, notamment sous forme supportée ou sous forme d'oxydes.

L'agent de réduction préférentiellement utilisé pour effectuer la réaction d'amination réductrice du composé de formule (VII) par le composé de formule (VIII) est le triacétoxyborohydrure de sodium.

Parmi les solvants pouvant être utilisés pour effectuer la réaction d'amination réductrice du composé de formule (VII) par le composé de formule (VIII), on peut citer à titre non limitatif le tétrahydrofurane, le dichlorométhane, le 1,2-dichloroéthane, les acétates, les alcools, préférentiellement l'éthanol, le méthanol ou l'isopropanol, le toluène et le xylène.

Dans un mode de réalisation préféré de l'invention, le solvant utilisé pour effectuer la réaction d'amination réductrice du composé de formule (VII) par le composé de formule (VIII) est constitué d'un mélange de solvants organiques.

Le solvant préférentiellement utilisé pour effectuer la réaction d'amination réductrice du composé de formule (VII) par le composé de formule (VIII) est constitué par un mélange de tétrahydrofurane et de dichlorométhane.

Le composé de formule (VII), sous forme racémique ou optiquement active, est un produit nouveau, utile comme intermédiaire de synthèse dans l'industrie chimique ou pharmaceutique, notamment dans la synthèse de l'ivabradine, de ses sels d'addition à un acide pharmaceutiquement acceptable et de leurs hydrates, et fait à ce titre partie intégrante de la présente invention.

### Liste des abréviations utilisées :

DMF : *N*,*N*-diméthylformamide
THF : tétrahydrofurane
IR : infrarouge

Les exemples ci-dessous illustrent l'invention.

Les points de fusion (PF) ont été mesurés au banc Köfler.

### EXEMPLE 1 : 7,8-Diméthoxy-3-[3-(méthylamino)propyl]-1,3,4,5-tétrahydro-2H-3-benzazépin-2-one

Solubiliser 50 g (0,18 mole) de 3-(7,8-diméthoxy-2-oxo-1,2,4,5-tétrahydro-3*H*-3-benzazépin-3-yl)propanal dans 625 mL de méthanol. Refroidir cette solution à 0°C puis ajouter 62,5 mL (0,81 mole ; 4,5 équivalents) d'une solution aqueuse à 40 % de méthylamine. Agiter une heure à 0°C puis ajouter 7,5 g (0,2 mole ; 1,1 équivalent) de NaBH₄. Agiter 30 minutes à 0°C puis agiter 12 heures à température ambiante. Evaporer le méthanol. Le résidu est repris par une solution aqueuse d'acide chlorhydrique (1 N), lavé à l'acétate d'éthyle. La phase aqueuse est ensuite amenée à pH = 8 par addition de soude à 20% et extraite au dichlorométhane. La phase organique est lavée à l'eau, séchée sur MgSO₄, filtrée puis évaporée à sec pour obtenir 52 g d'une huile qui est purifiée par flash-chromatographie sur 1,5 kg de silice (éluant = dichlorométhane/éthanol/NH₄OH : 80/20/2). On obtient 42 g de produit attendu sous la forme d'un solide blanc.
Rendement = 80%
PF (BK) = 68-70°C

### EXEMPLE 2 : 7,8-Diméthoxy-3-[3-(méthylamino)propyl]-1,3-dihydro-2H-3-benzazépin-2-one

### Stade 1 : [3-(7,8-Diméthoxy-2-oxo-1,2-dihydro-3H-3-benzazépin-3-yl)propyl]méthyl carbamate de tert-butyle

Mettre en suspension 1,7 g (7,8 mmoles) de 7,8-diméthoxy 1,3-dihydro-2*H*-3-benzazépin-2-one dans 35 mL de DMF puis ajouter 374 mg (9,35 mmoles, 1,2 équivalents) d'hydrure de sodium (suspension à 60% dans l'huile). On obtient une solution jaune pâle limpide que l'on agite une heure à 25°C. On ajoute ensuite goutte à goutte 1,94 g (9,35 mmoles, 1,2 équivalent) de (3-chloropropyl)méthyl carbamate de *tert*-butyle en solution dans 10 mL de DMF. On chauffe à 50°C pendant une nuit puis on évapore à sec le solvant. Le résidu est repris à l'eau et extrait au dichlorométhane. La phase organique est séchée sur MgSO₄, filtrée puis évaporée à sec. On obtient 4,2 g d'une huile qui est purifiée par flash chromatographie sur 200g de silice (éluant = dichlorométhane/acétate d'éthyle : 80/20). On obtient alors 2,3 g de produit attendu sous forme d'huile incolore.
Rendement = 77%
IR (pur) : v= 1685, 1659, 1155, 1102, 872, 770 cm⁻¹.

### Stade 2 : 7,8-Diméthoxy-3-[3-(méthylamino)propyl] 1,3-dihydro-2H-3-benzazépin-2-one

Solubiliser dans 30 mL d'éthanol 1,9 g (4,86 mmoles) de produit obtenu au stade 1 et ajouter à cette solution 7 mL (24,3 mmoles, 5 équivalents) d' éthanol chlorhydrique (3,5 N). Chauffer une nuit à 60°C et évaporer à sec le milieu réactionnel. Le résidu obtenu est repris à l'eau, la phase aqueuse est ensuite amenée à pH=8 par addition de soude à 20% et extraite au dichlorométhane. La phase organique est lavée à l'eau, séchée sur MgSO₄, filtrée puis évaporée à sec. On obtient 1,1g de produit du titre sous forme d'une huile incolore.
Rendement = 78%
IR (pur) : ν= 3400, 1651, 1610, 1510, 856, 710 cm⁻¹.

### EXEMPLE 3 : 3,4-Diméthoxy-bicyclo[4.2.0]octa-1,3,5-triène-7-carbonitrile

Le composé du titre peut-être obtenu par cyclisation du 3-(2-bromo-4,5-diméthoxyphényl)propanenitrile comme décrit dans la littérature (voir Tetrahedron 1973, 29 73-76) ou selon l'enchaînement réactionnel suivant :

### Stade 1 : 3,4-Diméthoxy-6-hydroxy-benzaldéhyde

Dans un tricol, disperser sous agitation 40,8 g de chlorure d'aluminium sur 200mL de dichlorométhane. Refroidir la solution à 0°C. Couler goutte à goutte une solution de 20 g (0,101 mol) de 3,4,6-triméthoxybenzaldéhyde dissous dans 100 mL de dichlorométhane.

Laisser la température remonter à 19°C et agiter 45min. Hydrolyser le milieu réactionnel sur 400 g d'eau et de glace puis ajouter 100 mL d'HCl 1N et agiter 30 minutes. Laisser décanter puis extraire avec 200 mL de dichlorométhane. La phase organique est lavée avec 100mL d'HCl 1N, 100 mL d'eau et 100 mL de solution aqueuse saturée en NaCl, puis séchée sur MgSO₄, filtrée et évaporée à sec. On obtient 16,4 g de produit du titre.
Rdt = 77 %
IR (pur) : 1625, 1146 cm⁻¹.

### Stade 2 : 2-Formyl-4,5-diméthoxyphényl sulfamate de diméthyle

Dissoudre 16,2 g (0,0889 mol) de 3,4-diméthoxy-6-hydroxybenzaldéhyde dans 80 mL de DMF. Refroidir à 10°C et ajouter par portion 24,6 g (0,178 mol) de carbonate de potassium. Laisser remonter la température à l'ambiante et agiter 30 minutes. Refroidir à 10°C environ et couler goutte à goutte 10,1 mL (0,093mol) de chlorure de *N,N-*diméthylsulfamoyle. Laisser remonter la température à TA et agiter 2 h. Couler le milieu réactionnel sur 600 g d'eau et de glace et agiter 1 h à température ambiante. Le précipité formé est filtré et lavé avec 3 fois 50 mL d'eau puis séché sous vide. On obtient 21,3 g de produit du titre.
Rendement = 83 %
IR (pur) : 1670, 1278, 1150 cm⁻¹.

### Stade 3 : 2-(2-cyanovinyl)-4,5-diméthoxyphényl sulfamate de diméthyle

Ajouter par portion 3,1 g (0,0773 mol) d'hydrure de sodium sur une solution à 0°C de 11,9 mL (0,0736 mol) de cyanométhylphosphonate de diéthyle dans 400 mL de THF. Refroidir le mélange à -10°C et ajouter goutte à goutte une suspension de 21,3 g (0,0736 mol) de 2-formyl-4,5-diméthoxyphényl sulfamate de diméthyle dans 200 mL de THF. Agiter 30 minutes et hydrolyser sur 600 mL d'une solution de bicarbonate de sodium et d'eau (50/50) puis extraire la solution avec 2 fois 300 mL de toluène. La phase organique est lavée avec 100 mL d'eau et 100 mL de solution aqueuse saturée en NaCl puis séchée sur MgSO₄, filtrée et évaporée à sec. Le résidu obtenu est cristallisé dans 80 mL de diisopropyléther à température ambiante puis filtré et lavé avec 20 mL de diisopropyléther et séché sous vide. On obtient 18,4 g de produit du titre.
Rendement = 80 %
IR (pur) : 2217, 1361, 1165 cm⁻¹.

### Stade 4 : 2-(2-cyanoéthyl)-4,5-diméthoxyphényl sulfamate de diméthyle

Disperser 6,7 g (0,177 mol) de borohydrure de sodium dans 150 mL de THF. Couler goutte à goutte une suspension de 18,4 g (0,059 mol) de 2-(2-cyanovinyl)-4,5-diméthoxyphényl sulfamate de diméthyle dans 200 mL de THF. Couler goutte à goutte 48 mL de méthanol. Chauffer 3 h à 50°C puis refroidir et ajouter 1 g (0,026 mol) de borohydrure de sodium. Chauffer le milieu réactionnel à 50°C 1 h puis laisser agiter à température ambiante la nuit. Hydrolyser en coulant sur le milieu réactionnel 60 mL de solution aqueuse d'HCl 4N et en maintenant la température à environ 20°C. Ajouter 40 g de glace et 30 mL d'eau puis extraire avec 2 fois 200 mL d'acétate d'éthyle. La phase organique est lavée avec de l'eau et une solution aqueuse saturée en NaCl puis séchée sur MgSO4, filtrée et évaporée à sec. On obtient 17,4 g de produit du titre.
Rendement = 94 %
IR (pur) : 2246 cm⁻¹.

### Stade 5 : 3,4-Diméthoxybicyclo[4.2.0]octa-1,3,5-triène-7-carbonitrile

Mélanger sous azote 5,4 mL (0,0382 mol) de diisopropylamine redistillée et 60 mL de THF. Refroidir le mélange à -50°C et couler goutte à goutte 15,3 mL (0,0382 mol) de butyllithium 2.5N en solution dans l'hexane. Laisser remonter la température à -5°C et agiter 10 minutes. Refroidir la solution à -60°C et couler goutte à goutte une solution de 3 g (0.00954 mol) de 2-(2-cyanoéthyl)-4,5-diméthoxyphényl sulfamate de diméthyle dans 35 mL de THF. Laisser la température remonter lentement jusqu'à -24°C en contrôlant par HPLC la disparition du réactif. Additionner le milieu réactionnel sur un mélange d'eau et de glace et extraire avec de l'acétate d'éthyle. Laver les phases organiques successivement avec de la soude 1N, une solution aqueuse d'HCl 1N, de l'eau et une solution aqueuse saturée en NaCl puis les sécher sur MgSO₄, filtrer et évaporer les solvants. On obtient 2 g d'un résidu qui est purifié par flash chromatographie sur 70g de silice (éluant = dichlorométhane) pour conduire à 0,9 g de produit du titre sous forme d'un solide blanc.
Rendement = 50 %
PF (BK) = 89-91°C

### EXEMPLE 4 : (R,S)-3,4-Diméthoxy-bicyclo[4.2.0]octa-1,3,5-triène-7-carbaldéhyde

On solubilise 10g (52,8 mmoles) de 3,4-diméthoxy-bicyclo[4.2.0]octa-1,3,5-triène-7-carbonitrile dans 100 mL de toluène anhydre. On refroidit à -78°C puis on ajoute goutte à goutte 55 mL d'une solution d'hydrure de diisobutylaluminium (1,2 M dans le toluène) en maintenant la température inférieure à - 65°C (temps d'addition = 45 minutes). On laisse agiter 1 heure à -78°C après la fin de la coulée. On hydrolyse en ajoutant goutte à goutte 20 mL de méthanol. On laisse revenir la température à - 30°C puis on additionne le milieu réactionnel sur 200 mL d' HCl (0,1 N) et on extrait 2 fois à l'éther. La phase organique est lavée successivement à l'eau et par une solution aqueuse saturée de NaCl puis séchée sur MgSO₄, filtrée et évaporée à sec pour obtenir 8g de produit du titre sous forme d'huile jaune pâle.
Rendement = 79%
IR (pur) : ν = 2714, 2630, 1712 cm⁻¹.

### EXEMPLE 5 : (R,S)-3-(3-{[(3,4-diméthoxybicyclo[4.2.0]octa-1,3,5-trién-7-yl)méthyl] (méthyl)amino}propyl)-7,8-diméthoxy-1,3,4,5-tétrahydro-2H-3-benzazépin-2-one

On met en solution 8 g (52,4 mmoles, 1.2 équivalent) de (*R,S*)-3,4-diméthoxybicyclo[4.2.0]octa-1,3,5-triène-7-carbaldéhyde dans un mélange de 150 mL de THF anhydre et 20 mL de dichlorométhane. On ajoute 12,8 g (43,6 mmoles) de 7,8-diméthoxy-3-[3-(méthylamino)propyl]-1,3,4,5-tétrahydro-2*H*-3-benzazépin-2-one et 2,48 mL (43,6 mmoles) d'acide acétique. On refroidit à 0°C et on agite 30 minutes. Puis on ajoute 14 g (65,6 mmoles, 1.5 équivalent) de triacétoxyborohydrure de sodium. La réaction est instantanée. On évapore à sec. Puis le résidu est repris dans la soude 1N et extrait au dichlorométhane. La phase organique est lavée à l'eau, séchée sur MgSO₄, filtrée et évaporée à sec pour obtenir 20 g d'une huile qui est purifiée par flash-chromatographie sur 800 g de silice (éluant = dichlorométhane/éthanol/NH₄OH : 90/10/1). On obtient 16,8 g de produit du titre sous forme d'une huile incolore qui cristallise à température ambiante.
Rendement = 82%
PF (BK) = 98-100°C

### EXEMPLE 6 : Chlorhydrate du 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

2,1 g du composé racémique obtenu à l'exemple 5 sont séparés sur une colonne de 60 cm x 60 mm packée avec 2,1 kg de phase Chiralpak ® AD (granulométrie 20 µm). L'éluant utilisé est un mélange éthanol/acétonitrile/diéthylamine (10/90/0,1 en volume) à un débit de 50 mL/min. Le détecteur ultra-violet associé est utilisé à une longueur d'onde de 280 nm.
On obtient 0,95 g de l'énantiomère de configuration (R) sous forme de meringue blanche puis 0,95 g de l'énantiomère de configuration (*S*) également sous forme de meringue blanche.

Le chlorhydrate de l'énantiomère de configuration (*S*) est ensuite obtenu en suivant le mode opératoire décrit dans le brevet EP 0 534 859 (Exemple 2, stade E).

### EXEMPLE 7 : 3-{3-[[(3,4-diméthoxybicyclo[4.2.0]octa-1,3,5-trién-7-yl)methyl](méthyl)amino]propyl}7,8-diméthoxy-1,3-dihydro-2H-3-benzazépin-2-one

Solubiliser 1,1 g (3,78 mmoles) de 7,8-diméthoxy-3-[3-(méthylamino)propyl]1,3-dihydro-2*H*-3-benzazépin-2-one dans 50 mL de THF et 7 mL de dichlorométhane. Ajouter 0,69 g (4,53 mmoles, 1,2 équivalent) de 3,4-diméthoxybicyclo[4.2.0]octa-1,3,5-triène-7-carbaldéhyde et 0,22 ml d'acide acétique. Refroidir le milieu réactionnel à 0°C, et ajouter 1,2 g (5,67 mmoles, 1,5 équivalent) de triacétoxyborohydrure de sodium. La réaction est instantanée. Evaporer à sec. Le résidu est repris à l'eau, la phase aqueuse est amenée à pH=8 par addition de soude à 20% et extraite au dichlorométhane. La phase organique est lavée à l'eau, séchée sur MgSO₄, filtrée et évaporée à sec. On obtient 1,7 g d'une huile qui est purifiée par flash-chromatographie sur 100 g de silice (éluant = dichlorométhane/éthanol/NH₄OH : 95/5/0,5) pour conduire à 1,4 g de produit du titre sous forme d'une huile incolore.
Rendement = 79%
IR (pur) : ν= 1656, 1607, 1511, 1273, 1206, 1102, 836, 760 cm⁻¹.

### EXEMPLE 8 : Chlorhydrate du 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

1,4 g du composé racémique obtenu à l'exemple 7 sont séparés sur une colonne de 60 cm x 60 mm packée avec 3 kg de phase Chiralpak ® T101 (granulométrie 20 µm). L'éluant utilisé est un mélange éthanol/acétonitrile/diéthylamine (10/90/0,1 en volume) à un débit de 100 mL/min. Le détecteur ultra-violet associé est utilisé à une longueur d'onde de 280 nm.
On obtient 0,56 g de l'énantiomère de configuration (R) sous forme d'une huile incolore puis 0,62 g de l'énantiomère de configuration (*S*) également sous forme d'une huile incolore.

Le composé de configuration (*S*) est ensuite hydrogéné en suivant le mode opératoire décrit dans le brevet EP 0 534 859 (Exemple 1, stade D). Le chlorhydrate du composé obtenu est préparé en suivant le mode opératoire décrit dans le brevet EP 0 534 859 (Exemple 2, stade E).

### EXEMPLE 9 : Chlorhydrate du 3-{3-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

En opérant comme à l'exemple 5 à partir de (7*S*)-3,4-diméthoxy-bicyclo[4.2.0]octa-1,3,5-triène-7-carbaldéhyde et de 7,8-diméthoxy-3-[3-(méthylamino)propyl]-1,3,4,5-tétrahydro-2*H*-3-benzazépin-2-one, on obtient l'ivabradine base qui est ensuite transformée en son chlorhydrate en suivant le mode opératoire décrit dans le brevet EP 0 534 859 (Exemple 2, stade E).

### EXEMPLE 10 : Chlorhydrate du 3-13-[{[(7S)-3,4-diméthoxybicyclo[4.2.0]octa-1,3,5-trién-7-yl]méthyl}(méthyl)amino]propyl}-7,8-diméthoxy-1,3,4,5-tétrahydro-2H-3-benzazépin-2-one

En opérant comme à l'exemple 7 à partir de (7*S*)-3,4-diméthoxy-bicyclo[4.2.0]octa-1,3,5-triène-7-carbaldéhyde et de 7,8-diméthoxy-3-[3-(méthylamino)propyl]1,3-dihydro-2*H*-3-benzazépin-2-one, on obtient un composé qui est hydrogéné en suivant le mode opératoire décrit dans le brevet EP 0 534 859 (Exemple 1, stade D) pour conduire à l'ivabradine base, qui est ensuite transformée en son chlorhydrate en suivant le mode opératoire décrit dans le brevet EP 0 534 859 (Exemple 2, stade E).

## Revendications

1. Procédé de synthèse du composé de formule (VI), sous forme racémique ou optiquement active : 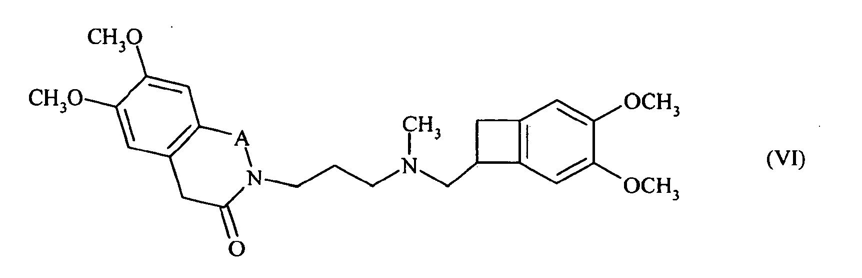 dans laquelle A représente H₂C-CH₂ ou HC=CH,
**caractérisé en ce que** l'on soumet le composé de formule (VII), sous forme racémique ou optiquement active : 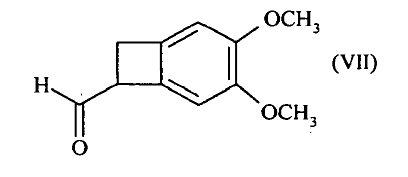 à une réaction d'amination réductrice par le composé de formule (VIII) : 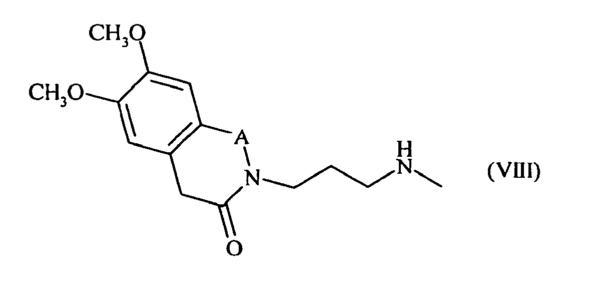 dans laquelle A est tel que défini précédemment,
en présence d'un agent de réduction,
dans un solvant organique ou un mélange de solvants organiques.

2. Procédé de synthèse selon la revendication 1, **caractérisé en ce que** le composé de formule (VII) est sous forme optiquement active et plus particulièrement de configuration (S).

3. Procédé de synthèse selon la revendication 2, **caractérisé en ce que** le groupement A représente H₂C-CH₂ et que le produit de la réaction d'amination réductrice du composé de formule (VII) par le composé de formule (VIII) est l'ivabradine de formule (I) : 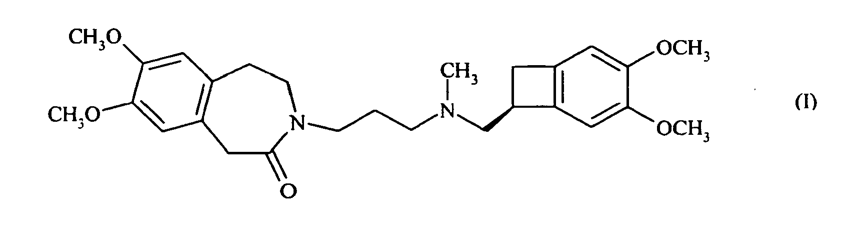 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

4. Procédé de synthèse selon la revendication 2, **caractérisé en ce que** le groupement A représente HC=CH et que le produit de la réaction d'amination réductrice du composé de formule (VII) par le composé de formule (VIII) est le composé de formule (V) : 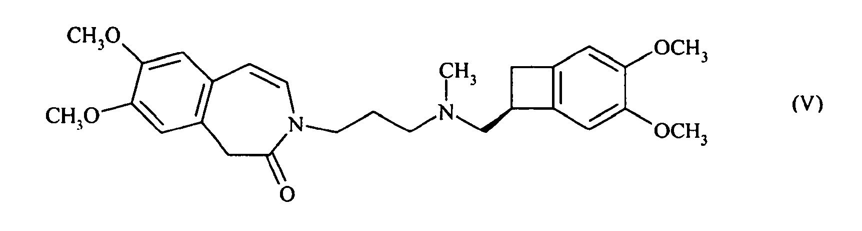 dont l'hydrogénation catalytique conduit à l'ivabradine de formule (1) : 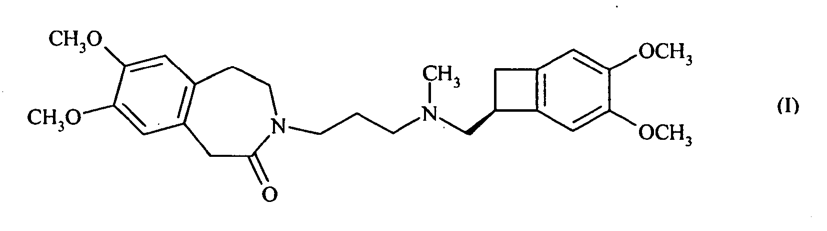 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

5. Procédé de synthèse selon la revendication 1, **caractérisé en ce que** le composé de formule (VII) est sous forme racémique et que la réaction d'amination réductrice du composé de formule (VII) par le composé de formule (VIII) est suivie d'une étape de résolution optique du composé racémique de formule (VI) obtenu.

6. Procédé de synthèse selon la revendication 5, **caractérisé en ce que** A représente H₂C-CH₂ et que le produit obtenu après l'étape de résolution optique du composé de formule (VI) est l'ivabradine de formule (I) : 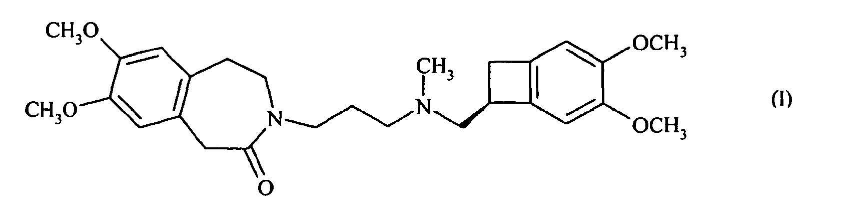 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

7. Procédé de synthèse selon la revendication 5, **caractérisé en ce que** A représente HC=CH et que le produit obtenu après l'étape de résolution optique du composé de formule (VI) est le composé de formule (V) : 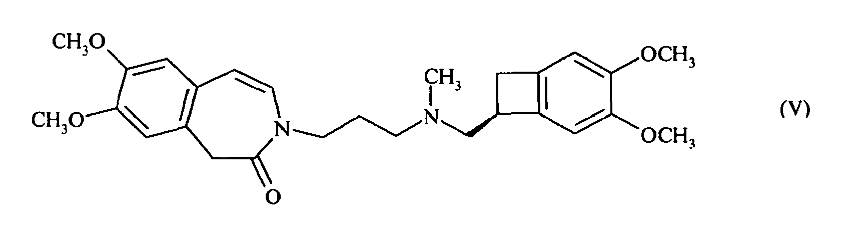 dont l'hydrogénation catalytique conduit à l'ivabradine de formule (I) : 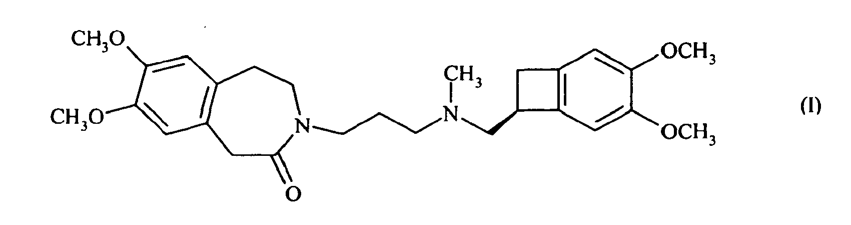 qui peut éventuellement être transformée en ses sels d'addition à un acide pharmaceutiquement acceptable, choisi parmi les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthanesulfonique, benzènesulfonique et camphorique, et en leurs hydrates.

8. Procédé de synthèse selon l'une quelconque des revendications 1 à 7, **caractérisé en ce que** l'agent de réduction utilisé pour effectuer la réaction d'amination réductrice du composé de formule (VII) par le composé de formule (VIII) est choisi parmi le triacétoxyborohydrure de sodium, le cyanoborohydrure de sodium ou le dihydrogène en présence d'un catalyseur tel que le palladium, le platine, le nickel, le ruthénium, le rhodium, ainsi que leurs dérivés, notamment sous forme supportée ou sous forme d'oxydes.

9. Procédé de synthèse selon la revendication 8, **caractérisé en ce que** l'agent de réduction utilisé pour effectuer la réaction d'amination réductrice du composé de formule (VII) par le composé de formule (VIII) est le triacétoxyborohydrure de sodium.

10. Procédé de synthèse selon l'une quelconque des revendications 1 à 9, **caractérisé en ce que** le solvant utilisé pour effectuer la réaction d'amination réductrice du composé de formule (VII) par le composé de formule(VIII) est choisi parmi le tétrahydrofurane, le dichlorométhane, le 1,2-dichloroéthane, les acétates, les alcools, préférentiellement l'éthanol, le méthanol ou l'isopropanol, le toluène ou le xylène.

11. Procédé de synthèse selon la revendication 10, **caractérisé en ce que** le solvant utilisé pour effectuer la réaction d'amination réductrice du composé de formule (VII) par le composé de formule (VIII) est constitué par un mélange de tétrahydrofurane et de dichlorométhane.

12. Composé de formule (VII) sous forme racémique ou optiquement active :

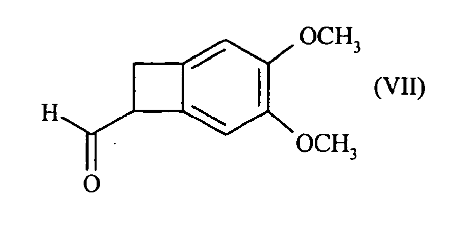

## Claims

1. Process for the synthesis of a compound of formula (VI) in racemic or optically active form: 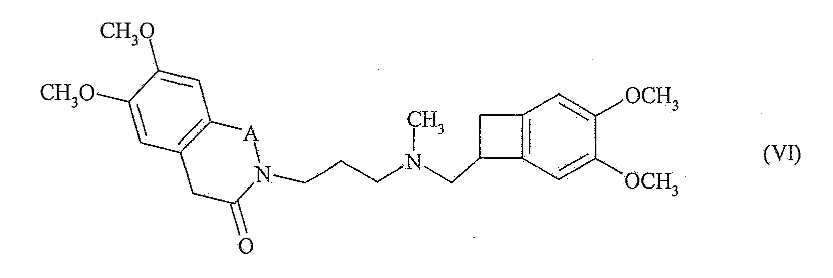 wherein A represents H₂C-CH₂ or HC=CH,
**characterised in that** the compound of formula (VII) in racemic or optically active form : 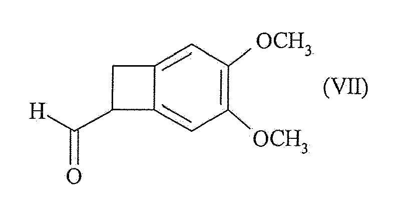 is subjected to a reductive amination reaction with a compound of formula (VIII) : 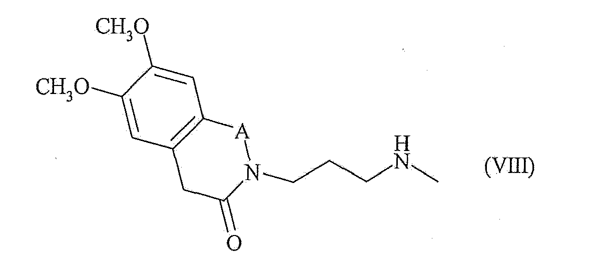 wherein A is as defined hereinbefore,
in the presence of a reducing agent,
in an organic solvent or a mixture of organic solvents.

2. Synthesis process according to claim 1, **characterised in that** the compound of formula (VII) is in optically active form and more especially has the (*S*) configuration.

3. Synthesis process according to claim 2, **characterised in that** the group A represents H₂C-CH₂ and the product of the reductive amination reaction of the compound of formula (VII) with a compound of formula (VIII) is ivabradine of formula (I) : 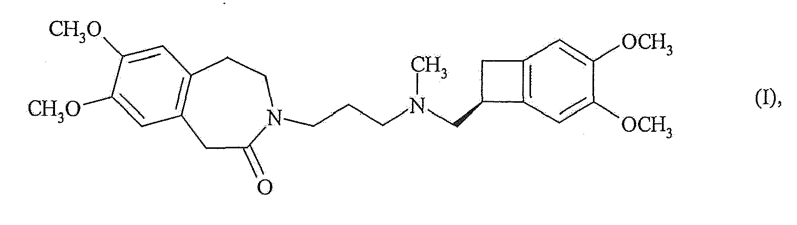 which may optionally be converted into its addition salts with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic, acid and camphoric acid, and into their hydrates.

4. Synthesis process according to claim 2, **characterised in that** the group A represents HC=CH and the product of the reductive amination reaction of the compound of formula (VII) with a compound of formula (VIII) is the compound of formula (V) : 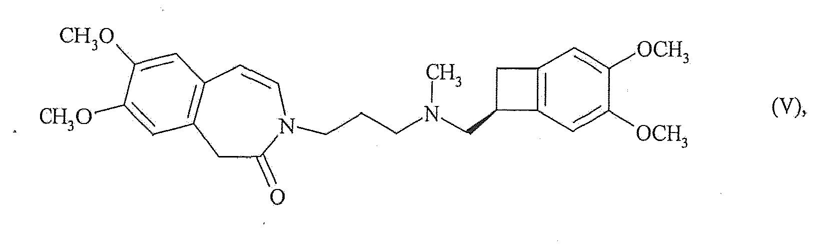 the catalytic hydrogenation of which yields ivabradine of formula (1) : 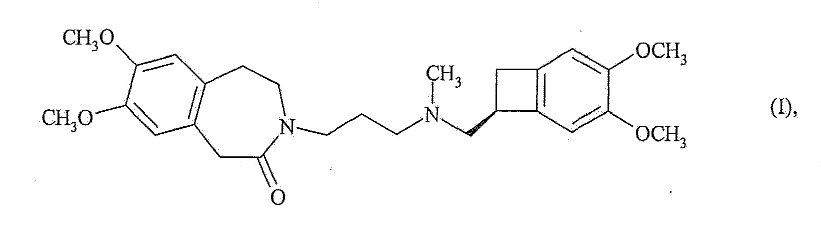 which may optionally be converted into its addition salts with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into their hydrates.

5. Synthesis process according to claim 1, **characterised in that** the compound of formula (VII) is in racemic form and the reductive amination reaction of the compound of formula (VII) with a compound of formula (VIII) is followed by an optical resolution step of the racemic compound of formula (VI) obtained.

6. Synthesis process according to claim 5, **characterised in that** A represents H₂C-CH₂ and the product obtained after the optical resolution step of the compound of formula (VI) is ivabradine of formula (I) : 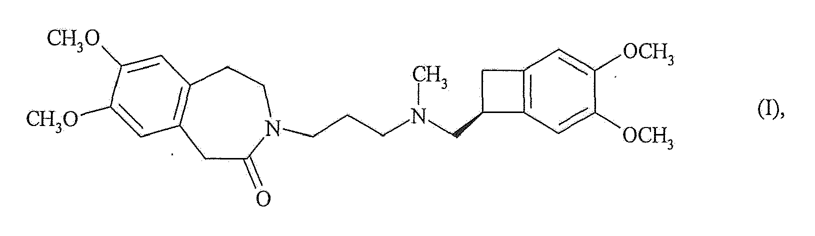 which may optionally be converted into its addition salts with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into their hydrates.

7. Synthesis process according to claim 5, **characterised in that** A represents HC=CH and the product obtained after the optical resolution step of the compound of formula (VI) is the compound of formula (V) : 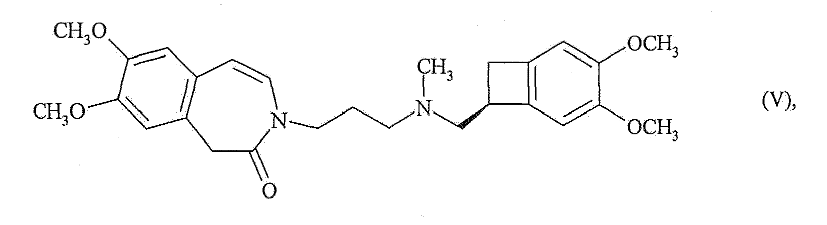 the catalytic hydrogenation of which yields ivabradine of formula (I): 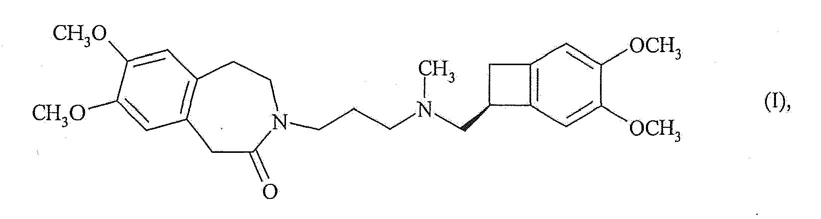 which may optionally be converted into its addition salts with a pharmaceutically acceptable acid selected from hydrochloric acid, hydrobromic acid, sulphuric acid, phosphoric acid, acetic acid, trifluoroacetic acid, lactic acid, pyruvic acid, malonic acid, succinic acid, glutaric acid, fumaric acid, tartaric acid, maleic acid, citric acid, ascorbic acid, oxalic acid, methanesulphonic acid, benzenesulphonic acid and camphoric acid, and into their hydrates.

8. Synthesis process according to any one of claims 1 to 7, **characterised in that** the reducing agent used to carry out the reductive amination reaction of the compound of formula (VII) with a compound of formula (VIII) is selected from sodium triacetoxyborohydride, sodium cyanoborohydride and dihydrogen in the presence of a catalyst such as palladium, platinum, nickel, ruthenium, rhodium, and derivatives thereof, especially in supported form or in oxide form.

9. Synthesis process according to claim 8, **characterised in that** the reducing agent used to carry out the reductive amination reaction of the compound of formula (VII) with a compound of formula (VIII) is sodium triacetoxyborohydride.

10. Synthesis process according to any one of claims 1 to 9, **characterised in that** the solvent used to carry out the reductive amination reaction of the compound of formula (VII) with a compound of formula (VIII) is selected from tetrahydrofuran, dichloromethane, 1,2-dichloroethane, acetates, alcohols, preferably ethanol, methanol or isopropanol, toluene and xylene.

11. Synthesis process according to claim 10, **characterised in that** the solvent used to carry out the reductive amination reaction of the compound of formula (VII) with a compound of formula (VIII) is constituted by a mixture of tetrahydrofuran and dichloromethane.

12. Compound of formula (VII) in racemic or optically active form :

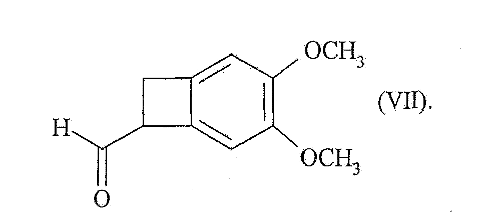

## Patentansprüche

1. Verfahren zur Synthese der Verbindung der Formel (VI) in racemischer oder optisch aktiver Form: 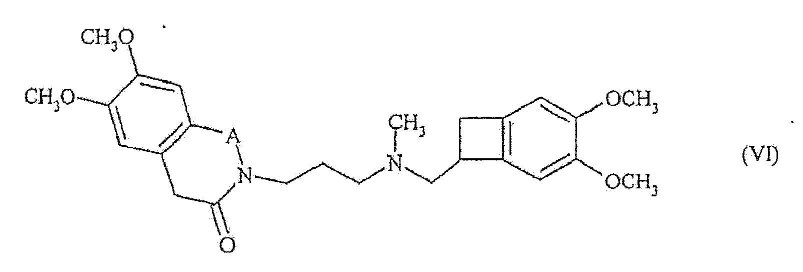 in der A H₂C-CH₂ oder HC=CH bedeutet,
**dadurch gekennzeichnet, dass** man die Verbindung der Formel (VII) in racemischer oder optisch aktiver Form: 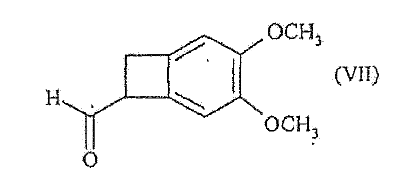 einer reduzierenden Aminierungsreaktion mit der Verbindung der Formel (VIII): 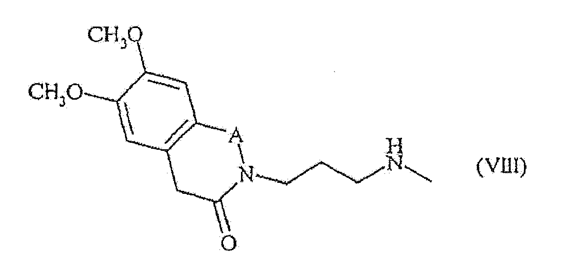 in der A die oben angegebenen Bedeutungen besitzt,
in Gegenwart eines Reduktionsmittels
in einem organischen Lösungsmittel oder einer Mischung von organischen Lösungsmitteln unterwirft.

2. Syntheseverfahren nach Anspruch 1, **dadurch gekennzeichnet, dass** die Verbindung der Formel (VII) in optisch aktiver Form vorliegt und insbesondere in der Konfiguration (S).

3. Syntheseverfahren nach Anspruch 2, **dadurch gekennzeichnet, dass** die Gruppe A H₂C-CH₂ bedeutet und dass das Produkt der reduzierenden Aminierungsreaktion der Verbindung der Formel (VII) mit der Verbindung der Formel (VIII) Ivabradin der Formel (I) ist: 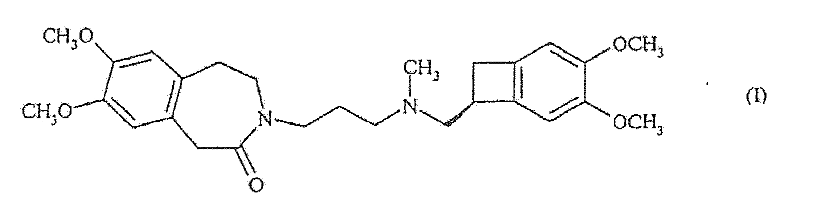 welches gegebenenfalls in eines seiner Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure und in ihre Hydrate umgewandelt werden kann.

4. Syntheseverfahren nach Anspruch 2, **dadurch gekennzeichnet, dass** die Gruppe A HC=CH bedeutet und dass das Produkt der reduzierenden Aminierungsreaktion der Verbindung der Formel (VII) mit der Verbindung der Formel (VIII) die Verbindung der Formel (V) ist: 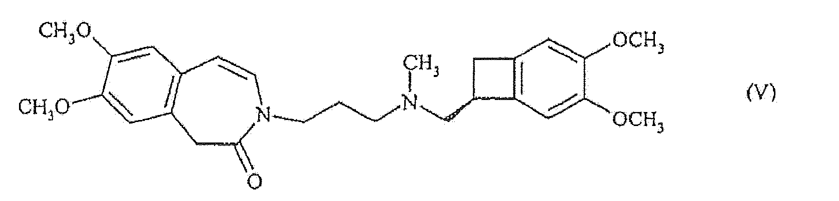 deren katalytische Hydrierung zu Ivabradin der Formel (I) führt: 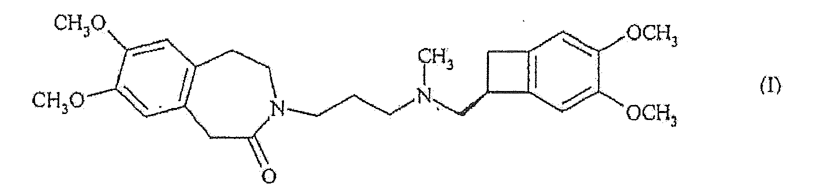 welches gegebenenfalls in eines seiner Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure und in ihre Hydrate umgewandelt werden kann.

5. Syntheseverfahren nach Anspruch 1, **dadurch gekennzeichnet, dass** die Verbindung der Formel (VII) in racemischer Form vorliegt und die reduzierende Aminierungsreaktion der Verbindung der Formel (VII) mit der Verbindung der Formel (VIII) gefolgt wird von einer Stufe der Racemattrennung der erhaltenen racemischen Verbindung der Formel (VI).

6. Syntheseverfahren nach Anspruch 5, **dadurch gekennzeichnet, dass** A H₂C-CH₂ bedeutet und dass das nach der Stufe der Racemattrennung der Verbindung der Formel (VI) erhaltene Produkt Ivabradin der Formel (I) ist: 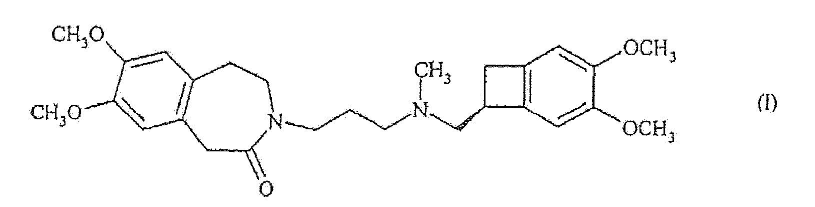 welches gegebenenfalls in eines seiner Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure und in ihre Hydrate umgewandelt werden kann.

7. Syntheseverfahren nach Anspruch 5, **dadurch gekennzeichnet, dass** A HC=CH bedeutet und dass das nach der Stufe der Racemattrennung der Verbindung der Formel (VI) erhaltene Produkt die Verbindung der Formel (V) ist: 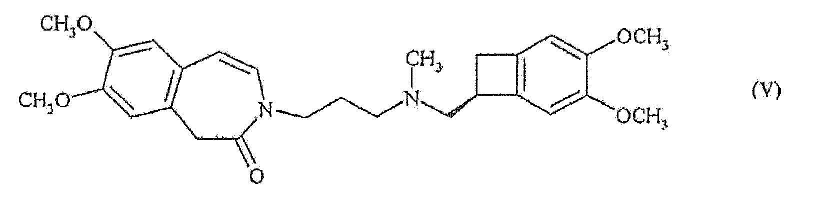 deren katalytische Hydrierung zu Ivabradin der Formel (I) führt: 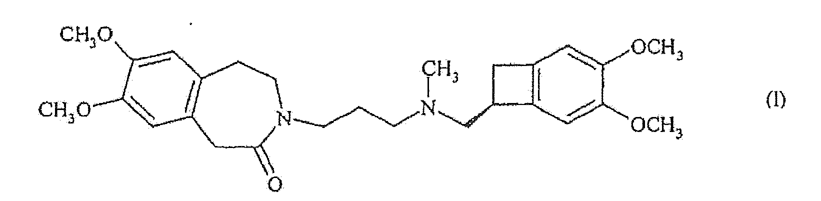 welches gegebenenfalls in eines seiner Additionssalze mit einer pharmazeutisch annehmbaren Säure ausgewählt aus Chlorwasserstoffsäure, Bromwasserstoffsäure, Schwefelsäure, Phosphorsäure, Essigsäure, Trifluoressigsäure, Milchsäure, Brenztraubensäure, Malonsäure, Bernsteinsäure, Glutarsäure, Fumarsäure, Weinsäure, Maleinsäure, Citronensäure, Ascorbinsäure, Oxalsäure, Methansulfonsäure, Benzolsulfonsäure und Camphersäure und in ihre Hydrate umgewandelt werden kann.

8. Syntheseverfahren nach einem der Ansprüche 1 bis 7, **dadurch gekennzeichnet, dass** das zur Durchführung der reduzierenden Aminierungsreaktion der Verbindung der Formel (VII) mit der Verbindung der Formel (VIII) verwendete Reduktionsmittel ausgewählt ist aus Natriumtriacetoxyborhydrid, Natriumcyanborhydrid oder Wasserstoff in Gegenwart eines Katalysators wie Palladium, Platin, Nickel, Ruthenium, Rhodium sowie deren Derivate, insbesondere in auf einem Träger vorliegender Form oder in Form von Oxiden.

9. Syntheseverfahren nach Anspruch 8, **dadurch gekennzeichnet, dass** das zur Durchführung der reduzierenden Aminierungsreaktion der Verbindung der Formel (VII) mit der Verbindung der Formel (VIII) verwendete Reduktionsmittel Natriumtriacetoxyborhydrid ist.

10. Syntheseverfahren nach einem der Ansprüche 1 bis 9, **dadurch gekennzeichnet, dass** das zur Durchführung der reduzierenden Aminierungsreaktion der Verbindung der Formel (VII) mit der Verbindung der Formel (VIII) verwendete Lösungsmittel ausgewählt ist aus Tetrahydrofuran, Dichlormethan, 1,2-Dichlorethan, Acetaten, Alkoholen, vorzugsweise Ethanol, Methanol oder Isopropanol, Toluol und Xylol.

11. Syntheseverfahren nach Anspruch 10, **dadurch gekennzeichnet, dass** das zur Durchführung der reduzierenden Aminierungsreaktion der Verbindung der Formel (VII) mit der Verbindung der Formel (VIII) verwendete Lösungsmittel durch eine Mischung aus Tetrahydrofuran und Dichlormethan gebildet wird.

12. Verbindung der Formel (VII) in racemischer oder optisch aktiver Form:
